(19) Europäisches Patentamt
European Patent Office
Office européen des brevets

(11) Numéro de publication: **0 378 022 A1**

(12) # DEMANDE DE BREVET EUROPEEN

(21) Numéro de dépôt: **89403550.0**

(22) Date de dépôt: **19.12.89**

(51) Int. Cl.5: **C07C 69/63, C07C 53/50, C07C 51/58, C07C 67/307**

(30) Priorité: **27.12.88 FR 8817241**

(43) Date de publication de la demande: **18.07.90 Bulletin 90/29**

(84) Etats contractants désignés: **BE DE FR GB IT**

(71) Demandeur: **ATOCHEM**
**4 & 8, Cours Michelet La Défense 10**
**F-92800 Puteaux(FR)**

(72) Inventeur: **Correia, Yves**
**Les Lauzières**
**F-04160 Chateau-Arnoux(FR)**
Inventeur: **Drivon, Gilles**
**4 Rue Joany Courbière**
**F-69850 Saint Martien en Haut(FR)**
Inventeur: **Lesparre, Jean**
**Quartier Savin**
**F-04290 Volonne(FR)**

(74) Mandataire: **Leboulenger, Jean et al**
**ATOCHEM Département Propriété Industrielle Cédex 42- La Défense 10**
**F-92091 Paris la Défense(FR)**

(54) **Composés chlorofluorés fonctionnels et leur préparation.**

(57) L'invention a pour objet des composés chlorofluorés fonctionnels de formule générale :

$$R_F\text{-}CCl_2\text{-}(CCl_2\text{-}O)_n\text{-}\overset{O}{\overset{\|}{C}}(X)_nY_{1\text{-}n} \qquad (I)$$

dans laquelle $R_F$ représente un radical perfluoroalkyle, n est égal à 0 ou 1, X désigne un atome de chlore ou de fluor ou un radical trichlorométhyle ou trifluorométhyle et Y représente un atome de chlore, un groupement hydroxyle éventuellement salifié ou estérifié ou un groupement amino eventuellement substitué, l'enchaînement $R_F\text{-}CCl_2\text{-}(CCl_2\text{-}O)_n\text{-}$ contenant au moins 3 atomes de carbone.

Par photochloration des esters $R_F\text{-}CH_2CH_2\text{-}O\text{-}CO\text{-}X$, on obtient les composés I (n = 1) qui sont ensuite réarrangés en chlorures d'acides I (n = 0, Y = Cl) à partir desquels on forme éventuellement les acides, sels, esters et amides correspondants (formule I avec n = 0 et Y = hydroxyle éventuellement salifié ou estérifié ou amino éventuellement substitué).

EP 0 378 022 A1

# COMPOSES CHLOROFLUORES FONCTIONNELS ET LEUR PREPARATION

La présente invention concerne le domaine des composés organiques polyhalogénés et a plus particulièrement pour objet des composés chlorofluorés fonctionnels dans lesquels un radical perfluoré est relié à un groupe fonctionnel acide ou dérivé (halogénure, ester, amide) par un ou deux ponts $-CCl_2-$.

De nombreux acides polyfluoro-carboxyliques, notamment les acides perfluoroalcane-carboxyliques et les acides (perfluoroalkyl)-2 éthane carboxyliques, sont déjà connus. Ces acides et leurs dérivés ont trouvé de nombreuses applications, par exemple comme agents mouillants ou tensio-actifs ou comme intermédiaires pour la préparation d'agents de traitement des textiles, du cuir ou du papier.

On connait également des acides carboxyliques chlorofluorés, mais dans la plupart le chlore et le fluor sont fixés sur les mêmes atomes de carbone comme, par exemple, dans les acides $Cl(CF_2CFCl)_n-COOH$ décrits par R.N. Haszeldine, J. Chem. Soc. 1955, 4291-4302, ou un pont $-CCl_2-$ interrompt la chaîne perfluorée comme, par exemple, dans l'acide $CF_3-CCl_2-CF_2-COOH$ décrit par B. Boutevin et al, Eur. Polym. J. 1976, 12(5) 283-8.

Cependant, ne sont actuellement connus que quelques composés dans lesquels le groupe fonctionnel acide est lié à un radical perfluoré par un pont $-CCl_2-$. Il s'agit plus précisement de l'acide dichloro-2,2 trifluoro-3,3,3 propionique, de son chlorure et de ses esters alkyliques (J.D. Park et al, J. Org. Chem. 21 (1956), 220-2) préparés à partir du dichloro-2,2 trifluoro-3,3,3 propényl éthyl éther. Le fluorure de cet acide, utilisable pour la préparation de tensio-actifs, d'agents d'imprégnation ou d'herbicides, a également été décrit dans le brevet DE 1 900 758 où il est préparé par action du fluorure de sodium ou de potassium sur l'hexachloroacétone.

La présente invention a maintenant pour objet une nouvelle série de composés chlorofluorés fonctionnels dans lesquels le groupe fonctionnel acide est lié à un radical perfluoré par un ou deux ponts $-CCl_2-$.

Les nouveaux composés selon l'invention peuvent être représentés par la formule générale :

$$R_F-CCl_2-(CCl_2-O)_n-\overset{\overset{O}{\|}}{C}(X)_nY_{1-n} \qquad (I)$$

dans laquelle $R_F$ représente un radical perfluoroalkyle, n est égal à 0 ou 1, X représente un atome de chlore ou de fluor ou un radical trichlorométhyle ou trifluorométhyle, et Y représente un atome de chlore, un groupement hydroxyle éventuellement salifié ou estérifié ou un groupement amino éventuellement mono- ou disubstitué, l'enchaînement $R_F-CCl_2-(CCl_2-O)_n-$ contenant au moins 3 atomes de carbone.

Le radical perfluoroalkyle $R_F$ peut être linéaire ou ramifié et contenir de 1 à 20 atomes de carbone. De préférence, il s'agit d'un radical linéaire contenant de 2 à 12 atomes de carbone.

Quand Y représente un groupement hydroxyle salifié, le cation peut être de nature minérale (métal alcalin, ammonium) ou organique (sel d'amines primaires, secondaires ou tertiaires).

Quand Y est un groupement hydroxyle estérifié, le radical alcoolique est avantageusement celui d'un alcool aliphatique primaire, secondaire ou tertiaire ou cycloaliphatique. On préfère les alcools contenant de 1 à 12 atomes de carbone tels que, par exemple, le méthanol, l'éthanol, l'éthyl-2 hexanol, le dodécanol et le cyclohexanol.

Quand Y est un groupement amino, celui-ci peut être substitué par des radicaux alkyle (de préférence en $C_1$ à $C_{12}$), cycloalkyle (par exemple cyclohexyle), aromatiques (par exemple phényle éventuellement substitué par un ou plusieurs atomes d'halogène, radicaux alkyle ou groupes nitro) ou hétérocycliques.

Les composés selon l'invention sont préparés suivant des méthodes connues en soi à partir des esters de formule :

$$R_F-CH_2CH_2-O\underset{\underset{O}{\|}}{C}-X \qquad (II)$$

dans laquelle $R_F$ et X ont la même signification que ci-dessus. Ces esters sont pour la plupart des produits connus qui, d'une façon générale, peuvent être obtenus par réaction d'un tétrahydro-1,1,2,2 perfluoroalcanol : $R_F-CH_2CH_2OH$ avec un halogénure d'acide : Hal-CO-X tel que le phosgène, le fluorure de carbonyle, le chlorure de trifluoroacétyle et, de préférence, le chlorure de trichloroacétyle. Une autre voie d'accès aux esters (II) où X est $CCl_3$ consiste à soumettre à l'action du peroxyde d'hydrogène un mélange d'iodure de (perfluoroalkyl)-2 éthyle : $R_F-CH_2CH_2I$, d'acide trichloroacétique et d'acide sulfurique à une température de 60 à 90 °C.

Par photochloration des esters de formule (II), on obtient les composés I-a (formule I avec n = 1), suivant la réaction :

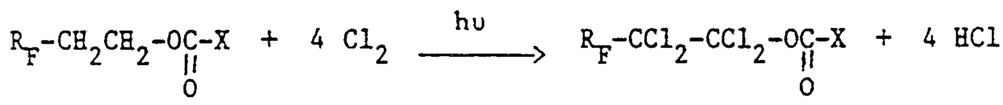


(I-a)

Cette photochloration peut être effectuée à une température comprise entre 50 et 200 °C, de préférence entre 80 et 170 °C, avec toute source lumineuse capable d'exciter l'atome de chlore, c'est-à-dire toute source émettant dans le bleu, par exemple des lampes à vapeur de mercure basse ou haute pression. L'opération peut être conduite en présence ou non d'un solvant inerte vis-à-vis du chlore, par exemple le tétrachlorure de carbone, les chlorobenzènes (notamment dichloro- et trichloro-benzènes) ou le chlorure de trichloroacétyle, le volume de solvant pouvant aller jusqu'à 10 fois celui du composé (II) de départ. Bien qu'on puisse opérer sous pression (jusqu'à 5 bars), la photochloration est habituellement effectuée à la pression atmosphérique ou mieux sous légère dépression. Le chlore gazeux qui doit contenir moins de 1000 ppm d'oxygène, mais peut être dilué avec un gaz inerte tel que l'azote, est introduit en continu dans le milieu réactionnel en une quantité qui peut varier dans de larges limites, mais est généralement comprise entre la quantité théorique (4 moles) et 10 fois cette quantité théorique. L'évolution de la photochloration peut être suivie par le dosage de l'acide chlorhydrique dégagé.

Les composés (I-a) peuvent, si on le désire, être isolés par distillation sous vide du mélange réactionnel et éventuellement purifiés par recristallisation dans un solvant inerte tel que, par exemple, l'hexane.

Par réarrangement des composés I-a (isolés ou non), on accède ensuite aux chlorures d'acide I-b (formule I avec n = 0 et Y = Cl) suivant la réaction :

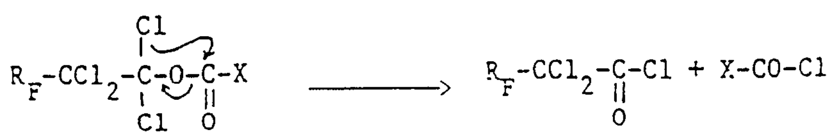

(I-a) (I-b)

Ce réarrangement s'effectue en général par chauffage à une température allant de 60 à 140 °C, de préférence comprise entre 80 et 120 °C. Pour accélérer l'opération on peut utiliser comme catalyseur une amine tertiaire ou un sel (notamment un halohydrate) d'une telle amine en une quantité qui peut atteindre 5 % par rapport au poids de composé I-a et est, de préférence, comprise entre 0,1 et 1 %. Selon une modalité préférée, ce réarrangement peut être réalisé, sans isolement du composé I-a, directement sur le mélange réactionnel issu de l'étape de photochloration à condition toutefois, si cette étape a été effectuée en présence d'un solvant léger, de l'éliminer préalablement par distillation sous vide.

Les chlorures d'acide I-b et X-CO-Cl formés peuvent être séparés par distillation fractionnée. Le chlorure d'acide X-CO-Cl récupéré peut être réutilisé pour la synthèse des esters (II) de départ.

A partir des composés I-b, on obtient les autres composés selon l'invention (formule I avec n = 0 et Y = groupement OH éventuellement salifié ou estérifié ou groupement amino éventuellement substitué) par les méthodes classiques de saponification, de salification, d'estérification et d'amidation.

Les composés selon l'invention peuvent être utilisés dans de nombreux domaines, en particulier comme intermédiaires pour faire par fluoration des acides carboxyliques perfluorés connus ou, notamment sous forme de sels de lithium, comme agents tensioactifs dans les applications usuelles des tensioactifs fluorés, par exemple pour la polymérisation de certains monomères fluorés.

Les exemples suivants illustrent l'invention sans la limiter. Les pourcentages indiqués s'entendent en poids, sauf mention contraire. Le solvant utilisé pour les spectres RMN est le chloroforme deutérié $CDCl_3$.

**EXEMPLE 1**

A 95,6 g (0,263 mole) de tridécafluoro-octanol $C_6F_{13}CH_2CH_2OH$, on a ajouté en une demi-heure à 30 °C 47,8 g (0,263 mole) de chlorure de trichloroacétyle. Après chauffage à 60 °C pendant une heure, l'acide chlorhydrique formé est éliminé par un courant d'azote à 20 °C.

Le trichloroacétate de tridécafluoro-octyle ainsi obtenu a ensuite été chloré sous rayonnement UV et à une température de 80 à 130 °C au moyen d'un courant de chlore au débit d'environ 0,3 mole/heure

jusqu'à dégagement de la quantité théorique d'acide chlorhydrique (environ 17 heures).

Le mélange obtenu (164 g) a ensuite été distillé sous vide et on a recueilli successivement les fractions suivantes :

- 1ère fraction (≦ 20 °C sous 1467 Pa) : 20,4 g de chlorure de trichloroacétyle
- 2ème fraction (60 à 68 °C sous 1467 Pa) : 56 g de $C_6F_{13}CCl_2COCl$ brut (chlore total : 20,4 %)
- 3ème fraction (151 °C sous 1067 Pa) : 80,5 g de $C_6F_{13}CCl_2CCl_2OCOCCl_3$ (chlore total : 38,1 %)
- résidu : 7,1 g.

Après recristallisation de la 3ème fraction dans l'hexane, on a obtenu un solide blanc de point de fusion égal à 59 °C et dont le spectre RMN$^{13}$C est le suivant :

| | | | | | | | |
|---|---|---|---|---|---|---|---|
| | $CF_3(CF_2)_5$ - | $CCl_2$ - | $CCl_2$ - | O - | C(=O) - | $CCl_3$ |
| δ(ppm) | .............. | 89,9 | | | 154,8 | 88,3 |

## EXEMPLE 2

A 96,2 g (0,207 mole) d'heptadécafluorodécanol $C_8F_{17}CH_2CH_2OH$, on a ajouté en une demi-heure à 45 °C 75 g de chlorure de trichloroacétyle, puis on a chauffé à 60 °C pendant une heure.

Après entraînement de l'acide chlorhydrique par un courant d'azote à 20 °C, le trichloroacétate d'heptadécafluorodécyle a été chloré entre 60 et 128 °C sous rayonnement UV avec un courant de chlore au débit d'environ 0,35 mole/heure jusqu'à ce qu'on ait recueilli sensiblement la quantité théorique d'acide chlorhydrique (environ 20 heures).

Le mélange a ensuite été distillé sous vide et on a obtenu successivement :

- une première fraction (< 92 °C sous 1333 Pa) constituée majoritairement par du chlorure de trichloroacétyle
- une deuxième fraction (92 °C sous 1333 Pa) pesant 77,8 g et constituée par le chlorure de dichloro-2,2 heptadécafluorodécanoyle $C_8F_{17}CCl_2COCl$ (chlore total : 18,8 %)
- un résidu pesant 31 g et ayant une teneur en chlore de 27,7 %.

Par recristallisation de ce résidu dans l'hexane, on a obtenu le trichloroacétate de tétrachloro-1,1,2,2 heptadécafluorodécyle $C_8F_{17}CCl_2CCl_2OCOCCl_3$ qui se présente sous forme d'un solide blanc de point de fusion égal à 62 °C (teneur en chlore : 32,3 %).

## EXEMPLE 3

*a/ Synthèse du chlorure de dichloro-2,2 tridécafluoro-octanoyle*

Dans un réacteur équipé d'une agitation, d'une entrée de gaz, d'une ampoule à brome, d'un réfrigérant relié à une colonne d'abattage à l'eau et d'un dispositif d'éclairement par une lampe bleue, on place 109,2 g (0,3 mole) de tridécafluoro-octanol $C_6F_{13}CH_2CH_2OH$, puis on porte à 60 °C et on coule en 15 minutes 54,6 g (0,3 mole) de chlorure de trichloroacétyle sous balayage d'azote : on recueille 0,29 mole d'acide chlorhydrique dans le gaz.

On introduit ensuite sous éclairement un courant de chlore (débit : 0,4 mole/heure) en montant la température jusqu'à 130-140 °C et on recueille les ions $Cl^-$ dégagés. Lorsque l'on a recueilli environ 1,2 mole de $Cl^-$ , on strippe le mélange par un courant d'azote, puis on ajoute 1 g de chlorhydrate de triéthylamine et on chauffe à 100 °C pendant 2 heures.

Par distillation sous vide (2533 Pa), on obtient d'abord du chlorure de trichloroacétyle, puis entre 83 et 85 °C 100 g de chlorure de dichloro-2,2 tridécafluoro-octanoyle $C_6F_{13}CCl_2COCl$ (Rendement : 71,6 %) identifié par les caractéristiques suivantes :

- Spectre de masse en impact électronique (ei) avec isotope (Cl = 35 : masse moléculaire = 464
- Spectre RMN $^{13}$C :

$\delta_{CO}$ = 162,6 ppm

$\delta_{CCl2}$ = 83,7 ppm

- Spectre RMN $^{19}F$ pour:

$$\begin{array}{lcccccccc} & CF_3 - & CF_2 - & CF_2 - & CF_2 - & CF_2 - & CF_2 & - CCl_2 & - COCl \\ \delta(ppm) = & 0 & 45,1 & 41,4 & 40,9 & 33,1 & 25,5 & & \end{array}$$

*b/ Acide dichloro-2,2 tridécafluoro-octanoïque $C_6F_{13}CCl_2COOH$*

Cet acide, obtenu par hydrolyse du chlorure de dichloro-2,2 tridécafluorooctanoyle, a été identifié par spectrométrie de masse en impact électronique (ei) avec l'isotope Cl = 35 : masse moléculaire = 446.

*c/ Ester méthylique de l'acide dichloro-2,2 tridécafluorooctanoïque*

Par réaction de l'acide précédent avec du méthanol en excès, on a obtenu l'ester méthylique correspondant $C_6F_{13}CCl_2COOCH_3$, identifié par les caractéristiques suivantes :
- Spectrométrie de masse en impact électronique : masse moléculaire = 460
- Spectre RMN $^1H$ (référence TMS) : $\delta$ = 3,97 ppm
- Spectre RMN $^{19}F$ pour:

$$\begin{array}{lcccccccc} & CF_3 - & CF_2 - & CF_2 - & CF_2 - & CF_2 - & CF_2 & - CCl_2 & - COOCH_3 \\ \delta(ppm) = & 0 & 45,3 & 41,5 & 41,0 & 34,7 & 27,4 & & \end{array}$$

*d/ N-(n.butyl) dichloro-2,2 tridécafluoro-octanamide*

Par réaction du chlorure de dichloro-2,2 tridécafluorooctanoyle avec la n.butylamine, on a obtenu le N-(n.butyl) dichloro-2,2 tridécafluoro-octanamide $C_6F_{13}CCl_2CONH(CH_2)_3CH_3$ dont le spectre IR est conforme (bande CO à 1695 $cm^{-1}$ ; bandes NH à 1520 et 3350 $cm^{-1}$ ; bande CF à 1200-1250 $cm^{-1}$).

*e/ Dichloro-2,2 tridécafluoro-octanoate de lithium*

A 15,4 g du chlorure de dichloro-2,2 tridécafluoro-octanoyle, on ajoute une solution de 2,78 g de lithine ($LiOH.H_2O$) dans 10g d'eau, puis on dilue le mélange avec de l'eau jusqu'à un poids d'environ 150 g. On obtient ainsi une solution à environ 10 % de dichloro-2,2 tridécafluoro-octanoate de lithium. La mesure de la concentration micellaire critique de ce sel de lithium, déterminée à partir cette solution à 10 %, est de $2,5.10^{-2}$ mole/litre.

A titre indicatif, cette concentration micellaire critique est, dans les mêmes conditions, de $2,4.10^{-2}$ mole/litre pour le perfluorooctanoate d'ammonium $C_7F_{15}COO^-NH_4^+$, produit commercial utilisé pour la polymérisation de certains monomères fluorés.

**EXEMPLE 4**

Dans un réacteur de 250 ml, on a introduit 68 g (0,41 mole) d'alcool pentafluorobutylique $C_2F_5CH_2CH_2OH$, puis en 15 minutes 75,5 g (0,41 mole) de chlorure de trichloroacétyle. On a chauffé le mélange à 60° C pendant 30 minutes. Après strippage par de l'azote, le produit a été distillé et on a recueilli 111,2 g de trichloroacétate de pentafluorobutyle (Eb 100° C sous 4000 Pa).

Cet ester a ensuite été photochloré entre 60 et 110° C pendant 4 heures avec 0,5 mole/h de chlore, puis pendant 10-12 heures à 140-145° C avec 0,3 mole/h de chlore. On a alors ajouté 1 g de chlorhydrate de triéthylamine et chauffé à 100° C pendant 2 heures.

Par distillation du mélange avec 13 plateaux réels, on a obtenu les trois fractions suivantes :

- 49 g à 108 °C sous 96,4 kPa : fraction constituée par du chlorure de dichloro-2,2 pentafluorobutanoyle ($C_2F_5CCl_2COCl$) brut,
- 10 g de fraction intermédiaire, et
- 31 g à 110-116 °C sous 96,4 kPa : fraction constituée essentiellement par du chlorure de trichloroacétyle.

Le chlorure de dichloro-2,2 pentafluorobutanoyle a été identifié par :

| | | | | | |
|---|---|---|---|---|---|
| - RMN $^{19}F$ pour | | $CF_3$ - | $CF_2$ - | $CCl_2$ - | COCl |
| | δ(ppm/TFA)) = | -1,9 | 34,5 | | |
| - RMN $^{13}C$ : | δ(ppm) = | 118,2 | 111,1 | 82,8 | 162,5 |

**EXEMPLE 5**

Dans le même appareillage qu'à l'exemple 3-a, on a introduit 193,8 g (0,443 mole) d'un mélange industriel d'alcools fluorés de formule : $C_nF_{2n+1}CH_2CH_2OH$ ayant la composition suivante :

| n | % |
|---|---|
| 6 | 49,3 |
| 8 | 30,5 |
| 10 | 13,5 |
| 12 | 5,7 |
| ≧ 14 | 1 |

Après addition de 120 g de chlorure de trichloroacétyle, on a chauffé à 60 °C pendant une heure, puis le mélange a été strippé avec de l'azote. Le produit a ensuite été photochloré pendant 6 heures à 120-130 °C avec 0,5 mole/h de chlore, puis pendant 14 heures à 150 °C avec 0,2 mole/h de chlore, l'acidité dégagée étant dosée dans l'évent gazeux.

On a ensuite ajouté 3 g de chlorhydrate de triéthylamine et le mélange a été maintenu à 100 °C pendant 2 heures avant de le distiller sous vide pour séparer les fractions suivantes :
- 1ère fraction (40 °C sous 4933 Pa) : chlorure de trichloroacétyle,
- 2ème fraction (de 62 °C sous 3333 Pa à 155 °C sous 2400 Pa) : 216 g d'un mélange brut des chlorures de formules: $C_nF_{2n+1}CCl_2COCl$ (chlore total : 24,9 %)
- résidu : 28 g.

**Revendications**

1. Composés chlorofluorés fonctionnels, caractérisés en ce qu'ils répondent à la formule générale :

$$R_F-CCl_2-(CCl_2-O)_n-\overset{O}{\overset{\|}{C}}(X)_nY_{1-n} \qquad (I)$$

dans laquelle $R_F$ représente un radical perfluoroalkyle, n est égal à 0 ou 1, X désigne un atome de chlore ou de fluor ou un radical trichlorométhyle ou trifluorométhyle, et Y représente un atome de chlore, un groupement hydroxyle éventuellement salifié ou estérifié ou un groupement amino éventuellement mono- ou disubstitué, l'enchaînement $R_F-CCl_2-(CCl_2-O)_n-$ contenant au moins 3 atomes de carbone.

2. Composés selon la revendication 1, dans lesquels $R_F$ est un radical linéaire contenant de 2 à 12 atomes de carbone.

3. Composés selon la revendication 1 ou 2, dans lesquels X est un radical trichlorométhyle.

4. Composés selon la revendication 1 ou 2, dans lesquels Y est un groupement hydroxyle salifié par une base minérale, l'ammoniac ou une amine primaire, secondaire ou tertiaire.

5. Composés selon la revendication 1 ou 2, dans lesquels Y est un groupement hydroxyle estérifié par un alcool aliphatique ou cycloaliphatique.

6. Composés selon la revendication 1 ou 2, dans lesquels Y est un groupement amino substitué ou non

par des radicaux alkyle, cycloalkyle, aromatiques ou hétérocycliques.

7. Procédé de préparation des composés de formule :

$R_F\text{-}CCl_2\text{-}CCl_2\text{-}O\underset{\overset{\|}{O}}{C}\text{ -}X$ (I-a)

dans laquelle $R_F$ et X sont tels que définis dans la revendication 1, caractérisé en ce que l'on soumet à une photochloration un ester de formule :

$R_F\text{-}CH_2\text{-}CH_2\text{-}O\underset{\overset{\|}{O}}{C}\text{ -}X$ (II)

8. Procédé de préparation des composés de formule :

$R_F\text{-}CCl_2\text{- }\underset{\overset{\|}{O}}{C}\text{-}Cl$ (I-b)

dans laquelle $R_F$ est tel que défini dans la revendication 1, caractérisé en ce que l'on chauffe un composé de formule :

$R_F\text{-}CCl_2\text{-}CCl_2\text{-}O\ \underset{\overset{\|}{O}}{C}\text{-}X$ (I-a)

à une température allant de 60 à 140° C, de préférence entre 80 et 120° C.

9. Procédé selon la revendication 8, dans lequel on opère en présence d'une amine tertiaire ou d'un sel d'une telle amine.

10. Procédé selon la revendication 8 ou 9, dans lequel on utilise un composé (I-a) brut tel qu'obtenu par photochloration selon la revendication 7.

Office européen des brevets

# RAPPORT DE RECHERCHE EUROPEENNE

Numero de la demande

EP 89 40 3550

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X,Y | US-A-3 047 610 (N.O. BRACE)<br>* Colonne 1, ligne 50 - colonne 2, ligne 5; colonne 4, lignes 56-74; colonnes 15-16; revendications *<br>--- | 1,2,5,7,8 | C 07 C 69/63<br>C 07 C 53/50<br>C 07 C 51/58<br>C 07 C 67/307 |
| Y | US-A-3 325 540 (L.G. ANELLO)<br>* Colonnes 6-7; revendications *<br>----- | 1,2 | |
| | | | **DOMAINES TECHNIQUES RECHERCHES (Int. Cl.5)** |
| | | | C 07 C 69/00<br>C 07 C 67/00<br>C 07 C 53/00<br>C 07 C 51/00 |

Le présent rapport a été établi pour toutes les revendications

| Lieu de la recherche | Date d'achèvement de la recherche | Examinateur |
|---|---|---|
| LA HAYE | 26-03-1990 | KINZINGER J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)